# EUROPÄISCHE PATENTSCHRIFT

(11) **EP 1 658 806 B1**
(45) Veröffentlichungstag und Bekanntmachung des Hinweises auf die Patenterteilung: **26.08.2009**
(21) Anmeldenummer: 04105835.5
(22) Anmeldetag: 17.11.2004
(51) Int. Cl.: A61B 5/1459

(54) **Kalibrierreflektorvorrichtung und damit ausgestattete Sondenanordnung**
Reflective calibration device and probe equipped with such device
Dispositif réflexif de calibration et sonde équipée avec ce dispositif

(43) Veröffentlichungstag der Anmeldung: 24.05.2006
(73) Patentinhaber: Pulsion Medical Systems AG, 81829 München (DE)
(72) Erfinder: Pfeiffer, Ulrich J. Dr., 81667 München (DE); Moulas, Daniel, 83539 Pfaffing (DE)
(74) Vertreter: Kehl, Günther

(56) Entgegenhaltungen:
- EP-A- 0 561 126
- US-A- 4 050 450
- US-A- 4 981 355
- US-A- 5 365 925

## Beschreibung

Die vorliegende Erfindung betrifft eine Kalibrierreflektorvorichtung für faseroptische Sonden sowie eine mit einer derartigen Kalibrierreflektorvorrichtung ausgestattete Sondenanordnung, insbesondere für die medizinische Anwendung.

Faseroptische Sonden sind an sich in vielfältiger Ausführung bekannt und werden in der Medizin beispielsweise zur intravasalen Messung der Sauerstoffsättigung im Blut eingesetzt. Um Infektionen mit oft schwerwiegenden Folgen bis hin zu einer tödlichen Sepsis zu vermeiden, muß der intravasale Teil der Sonde für die Anwendung unbedingt steril gehalten werden. Auch für andere, unter Umständen auch nichtmedizinische, in situ durchzuführende optische Messungen sind die an den Meßort zu führenden Sondenteile üblicherweise vor Kontamination zu schützen.

Um brauchbare Meßergebnisse zu erhalten, ist vor der Durchführung einer optischen Messung meist eine Sondenkalibrierung erforderlich. Hierfür werden üblicherweise Kalibrierreflektoren eingesetzt. Diese besitzen möglichst definierte Reflexionseigenschaften. Der Anteil des aus einer oder mehreren Emitterfasern der Sonde ausgesandten Lichts, welcher vom Kalibrierreflektor reflektiert und über die Meßfaser bzw. Meßfasern detektiert wird, dient als Bezugswert für die eigentlichen Messungen.

Kalibrierreflektorvorrichtungen und Sondenanordnungen der eingangs genannten Art sind u.a. aus den Druckschriften US 4,050,450 und EP 0 561 126 B1 bekannt.

Üblicherweise werden herkömmliche Kalibrierreflektorvorrichtungen an der Spitze der zu kalibrierenden Sonden festgeklemmt. Da die für die Sonden verwendeten Kunststoffe unter Last zum Kaltfließen neigen, führt die Klemmung insbesondere bei dünnen Sonden zu einer bleibenden Verformung. Diese Verformung stört sowohl das Einführen der Sonden durch Schleusen als auch die Messung. Ein weiteres Problem besteht darin, daß an die Klemmflächen kein Sterilisationsgas gelangen kann. Die Sonden müssen deshalb üblicherweise mit Gammastrahlen sterilisiert werden. Die eingesetzte Gammastrahlung verändert jedoch die optischen Eigenschaften der Sonden.

Vor diesem Hintergrund ist es Aufgabe der vorliegenden Erfindung, eine Kalibrierreflektorvorrichtung sowie eine damit ausgestattete Sondenanordnung zu schaffen, welche schädliche Verformungen der Sonde im Bereich der Spitze vermeidet und die Sterilisierbarkeit nicht beeinträchtigt. Darüberhinaus soll die Erfindung eine sichere und einfache Handhabung, insbesondere auch im klinischen Umfeld, gewährleisten. Ebenfalls erwünscht ist die Ausschaltung von Fremdlicht beim Kalibriervorgang. Ferner ist es Aufgabe der Erfindung, ein Verfahren zum sterilen Verpacken einer faseroptischen Sondenanordnung zu schaffen, welches ohne die Anwendung von Gammastrahlen einsetzbar ist.

Gemäß einem Aspekt der vorliegenden Erfindung wird die zugrundeliegende Aufgabe durch eine Kalibrierreflektorvorrichtung gemäß Anspruch 1 gelöst.

Vorteilhafte Ausführungsformen der erfindungsgemäßen Kalibrierreflektorvorrichtung können gemäß einem der Ansprüche 2-21 gestaltet sein.

Erfindungsgemäß ist für die empfindliche Sondenspitze also eine Art steife Hüllenanordnung in Form des Reflektorkörpers vorgesehen, welche die Kalibrationsmessung störende Knick- und Biegebelastungen weitgehend verhindert, wobei ein radiales Spiel vorgesehen ist, welche das Erreichen des intravasalen Sondenteils mit Sterilisiergas ermöglicht. Besonders vorteilhaft ist ein geringes radiales Spiel von unter 25%, vorzugsweise unter 10% des Sondendurchmessers im Bereich des intravasalen Teils, um den intravasalen Teil der Sonde gegen Verbiegen und Verkanten zu schützen.

Bei der vorzugsweise vorgesehenen Strukturierung der Oberfläche des Sondenhohlraums, beispielsweise in Form einer Aufrauhung und/oder Rippung der Oberfläche und/oder in Form eines sternförmigen und/oder anderweitig profilierten Hohlraumquerschnitts, kann die Verteilung des Sterilisiergases über die zu sterilisierenden Flächen oft noch verbessert werden. Außerdem werden dadurch störende Lichtspiegelungen an der Oberfläche vermieden. Eine Verbesserung der Verteilung des Sterilisiergases über die zu sterilisierenden Flächen läßt sich oft auch durch einen leicht ovalen Querschnitt des Sondenhohlraums bei rundem Sondenquerschnitt erzielen.

Der Terminus "intravasaler Teil" ist angesichts des avisierten Hauptanwendungszwecks gewählt, soll jedoch im Rahmen der vorliegenden Erfindung allgemein als der zum Meßort führende Teil einer faseroptischen Sonde verstanden werden, welcher für die Anwendung der Sonde vor Kontamination zu schützen bzw. zu sterilisieren ist.

Die Verbindung des Reflektorkörpers mit der Sonde über Anschlußmittel, welche radial außerhalb eines Freiraums angordnet sind, dessen Querschnitt genügend groß ist, um eine klemmfreie Anordnung des intravasalen Sondenteils zu ermöglichen, anstelle der herkömmlichen Klemmverbindung trägt ebenfalls zur Sterilisierbarkeit mittels Spülgas bei. Darüberhinaus wird durch den Verzicht auf Klemmstellen in der Nähe der Sondenspitze eine die Meßgenauigkeit beeinträchtigende Materialverformung vermieden. Realisierbar sind entsprechende Anschlußmittel beispielsweise als Verschraubung, etwa mittels handelsüblicher Luer-Lock-Muttern und -Gewinde, Bajonett-Anschluß, Klemmung außerhalb des intravasalen Sondenteils oder dergleichen.

Das vorzugsweise axial zwischen Anschlußmittel und Reflektorkörper vorgesehene Verlängerungsstück schützt den nicht im Sondenhohlraum befindlichen Bereich des intravasalen Sondenteils. Auch hier ist ein radiales Spiel (vorzugsweise etwas größer als das radiale Spiel im Sondenhohlraum) vorgesehen, um die Spühlung mit Sterilisiergas sowie das einfache Herausziehen der Sonde zu ermöglichen. Die innere Oberfläche des Verlängerungsstücks kann ähnlich gestaltet sein, wie im Sondenhohlraum. In vielerlei Hinsicht günstig kann die vorzugsweise schlauchartig flexible Gestaltung des Verlängerungsstücks sein.

Ist der Sondenhohlraum in einem flexiblen, schlauchartigen Reflektoreinsatz, beispielsweise aus Silikon oder Polyurethan, angeordnet, so kann dieser vorteilhafterweise in eine rohrartige Einhüllung und/oder einen ausreichend steifen Bereich der vorzugsweise vorgesehenen Blisterumhüllung (welche somit in der hier gewählten Terminologie auch als versteifende Einhüllung fungieren kann) eingebettet werden, um den steifen Reflektorkörper zu bilden und die erfindungsgemäße Vermeidung einer die Reflexionseigenschaften beeinflussenden Sondenbiegung zu gewährleisten.

Die Einhüllung wird möglichst aus einem zumindest für Licht bzw. Infrarot-Strahlung im (Wellenlängen-)Arbeitsbereich der Sonde undurchlässigen Material gefertigt.

Die vorzugsweise schwarze Ausführung der Einhüllung bietet einen sicheren Schutz gegen Streulichteinfall in den Reflektor sowie den dünnwandigen intravasalen Teil der Sonde.

Die steife Ausführung des Reflektorkörpers, das Spiel der Sonde im Sondenhohlraum sowie eine günstige Gestaltung der vorzugsweise vorgesehenen Blisterumhüllung erlauben auch das Herausziehen der Sonde aus dem Reflektorkörper mit geringem Kraftaufwand, ohne die Sonde zu knicken oder zu dehnen.

Um zu vermeiden, daß das Sondenende versehentlich vor der Kalibrierung aus dem Reflektorkörper gezogen wird, hat sich die Einstecktiefe von 20-60 mm, besonders bevorzugt 25-50 mm, als vorteilhaft erwiesen. Bevorzugt ist eine freie axiale Länge des Sondenhohlraums jenseits der Sondenspitze von mindestens dem zehnfachen, vorzugsweise mindestens dem zwanzigfachen der Wurzel der Querschnittsfläche des Sondenhohlraums bzw. des intravasalen Sondenteils. Bei einem Kreisquerschnitt ist dies etwas weniger als das zehn- bzw. zwanzigfache des Durchmessers.

Die vorzugsweise vorgesehene Blisterumhüllung gestattet auch die Implementierung eines besonders robusten, anwenderfreundlichen, selbsterklärenden und somit Anwenderfehler vermeidenden Systems.

Mit entsprechend gestalteten Ausführungsformen der Erfindung ist die Kalibrierprozedur und das Einführen der Sonde, beispielsweise in einen zentralvenösen Katheter, unter sterilen Bedingungen auch einer alleine hantierenden Person möglich.

So ist es besonders vorteilhaft für eine sterile Handhabung der Sonde nach dem Öffnen der Blister-Umhüllung und während der Kalibrierung, den vorzugsweise vorgesehenen Steckverbinder der Sonde unmittelbar im Bereich einer vorzugsweise vorgesehenen Aufreißlasche oder anderen Aufreißhilfe zu plazieren, und eine Markierung bzw. Begrenzungslinie vorzusehen, bis zu welcher die den Blister verschließende Folie, Faservlieslage (beispielsweise Tyvek von DuPont) oder dgl. aufzuziehen ist, um den Steckverbinder entnehmen zu können. Der restliche Teil der Sonde bleibt so noch von der Blisterumhüllung geschützt.

Um das, beispielsweise wie ein herkömmlicher Y-Konnektor ausführbare Anschlußstück vor dem Herausfallen zu schützen, kann vorteilhafterweise eine geeignete Klemmung oder Zugentlastung in der Blister-Umhüllung vorgesehen werden.

Besonders vorteilhaft kann ein zusätzliche Hülle sein, welche den steril zu haltenden Teil der Sonde, ggf. auch das Anschlußstück, selbst nach vollständiger Entnahme aus der vorzugsweise vorgesehenen Blisterumhüllung vor Kontamination schützt.

Gemäß einer vorteilhaften Weiterbidung der Erfindung kann die Sonde ein Lumen oder mehrere Lumina aufweisen und wie ein Katheter ausgebildet sein.

Ferner umfassen vorteilhafte Ausführungsformen der Erfindung nicht nur aber insbesondere auch Anordnungen mit besonders schlank ausgeführten Sonden, deren intravasaler Teil einen maximalen Querschnittsdurchmesser von 1 mm oder darunter aufweist.

Gemäß einem weiteren Aspekt der vorliegenden Erfindung wird die zugrundeliegende Aufgabe durch ein Verfahren gemäß Anspruch 22 gelöst.

Grundsätzlich kann jede im Rahmen der vorliegenden Anmeldung beschriebene bzw. angedeutete Variante der Erfindung besonders vorteilhaft sein, je nach wirtschaftlichen und technischen Bedingungen im Einzelfall. Soweit nichts gegenteiliges dargelegt ist, bzw. soweit grundsätzlich technisch realisierbar, sind einzelne Merkmale der beschriebenen Ausführungsformen austauschbar oder miteinander kombinierbar.

Nachfolgend werden anhand der zugehörigen Zeichnungen Beispiele bevorzugter Ausführungsformen der vorliegenden Erfindung näher erläutert. Die Zeichnungen sind dabei rein schematische und nicht maßstäbliche Darstellungen. Einander entsprechende Elemente sind in den einzelnen Figuren jeweils, soweit sinnvoll, mit denselben Bezugszeichen versehen.
- Fig. 1: zeigt als Längsschnitt eine erfindungsgemäße Sondenanordnung mit einer faseroptischen Sonde und einer erfindungsgemäßen Kalibrierreflektorvorrichtung, wobei die Darstellung im Bereich des als Schutzschlauch ausgeführten Verlängerungsstücks aus Gründen der Anschaulichkeit und des Zeichnungsblattformats unterbrochen ist.

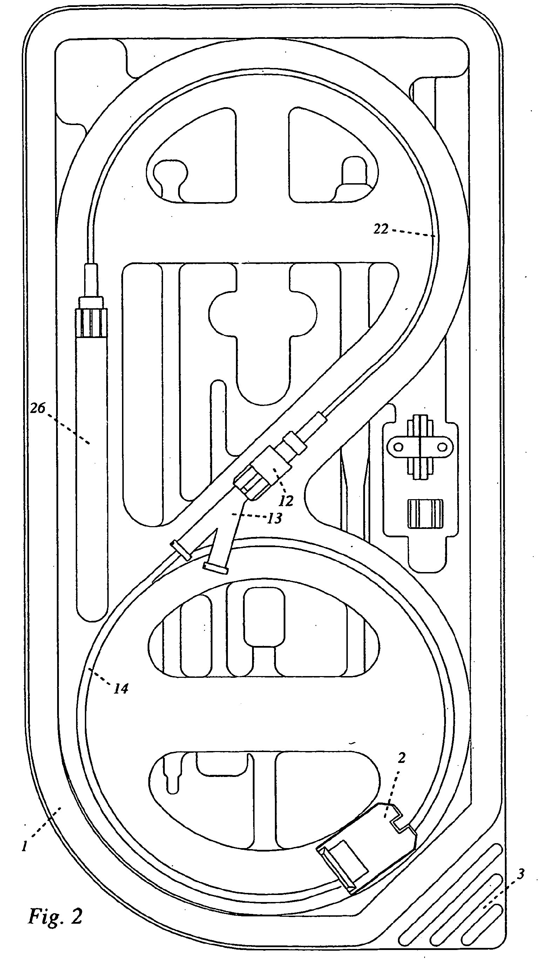

- Fig. 2: zeigt in der Draufsicht eine erfindungsgemäße Sondenanordnung mit aus Anschaulichkeitsgründen vollständig geöffneter Blisterverpackung, in welcher der extravasale Teil der Sonde als (sich in der Darstellung teilweise selbst überlappende) Schlaufe gelegt ist.

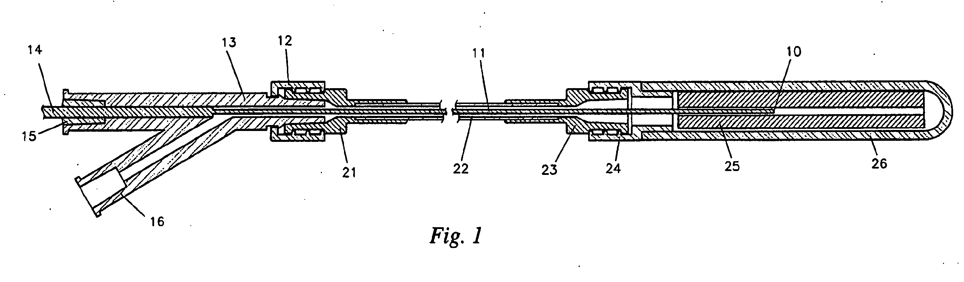

Wie in Fig. 1 im Längsschnitt dargestellt, befindet sich der schlauchartige Reflektoreinsatz 25 (hier aus extrudiertem Kunststoff z.B. Silicon oder Polyurethan mit einem Anteil von beispielsweise etwa 25% an Füllstoff wie etwa Bariumsulfat) lose in einer starren, lichtabsorbierenden, einseitig geschlossenen Hülse 26 und bildet mit dieser den zweiteiligen Reflektorkörper. An der offenen Seite der Hülse 26 ist eine Mutter 24 mit Luer-Lock Gewinde angeklebt. Der Reflektoreinsatz 25 ist dadurch eingeschlossen. Der steril zu haltende intravasale Teil der Sonde 11 ist in einem Schutzschlauch 22 mit beidseitig angeklebten Luer-Lock-Anschlußgewinden 21, 23 geführt. Dieser Schutzschlauch 22 ist mit den Muttern 12 und 24 an das Y-Anschlußstück 13 und die Reflektorhülse 26 angeschraubt (lösbare Luer Lock Verbindung).

Der verstärkte extravasale Teil 14 der Sonde ist mit Vergußmasse 15 in dem Y-Anschlußstück 13 dicht verklebt. Der Anschluß 16 kann für Infusionen und zum Spülen verwendet werden. Die Spitze der Sonde 10 steckt mit geringem Spiel (übertrieben dargestellt) im wesentlichen kräftefrei im Sondenhohlraum des Reflektoreinsatzes 25. Das Spiel stellt sicher, daß Sterilisationsgas an alle intravasalen Flächen gelangen kann. Durch eine rauhe Innenfläche oder einen profilierten Sondenhohlraum im Reflektoreinsatz 25 kann die Sterilisierbarkeit weiter verbessert werden. Solange die freie Länge im Sondenhohlraum mehr als das 20-fache dessen Innendurchmessers beträgt, ist die Einstecktiefe relativ unkritisch. Die Einstecktiefe wird durch die Länge des Schutzschlauchs 22 bestimmt. Die Befestigung des Reflektors an der Sonde erfolgt über die Verschraubung 12,21 ausserhalb des intravasalen Bereichs. Die Lage der Kalibrierreflektoreinrichtung (Schutzschlauch 22, Reflektorkörper 25, 26) relativ zur Sonde wird fixiert ohne schädliche Kräfte auf die Sonde auszuüben: Die Verschraubung 12, 21 sorgt für eine Verbindung der Kalibrierreflektoreinrichtung mit der Sonde, ohne daß der intravasale Sondenteil 11 einer Klemmbelastung ausgesetzt wäre. An allen intravasalen Flächen der Sonde ist ein freier Zugang von Sterilisationsgas möglich.

Zwischen Reflektoreinsatz 25 und Hülse 26 kann in einem Teilbereich des Umfangs ein Zwischenraum vorgesehen sein, um Sterilisationsgas den Eintritt in den Endbereich der Hülse 26 zu erleichtern, so daß das Gas von zwei Seiten her in den Sondenhohlraum eintreten kann. Dies läßt sich einfach beispielsweise durch eine innen in der Hülse und/oder außen im Reflektoreinsetz 25 vorgesehene Nut realisieren.

Die Sonde wird mit Schutzschlauch 22 und Reflektorkörper montiert, in einer Blisterumhüllung 1 verpackt und gassterilisiert. Nach dem Öffnen der Blisterverpackung 1 wird die Sonde über einen am extravasalen Teil 14 angebrachten Steckverbinder 2, welcher im verpackten Zustand nahe der Aufreißlasche 3 in den Blister 1 geklemmt ist, an ein Auswertegerät (nicht dargestellt) angeschlossen und mit der Kalibrierreflektorvorrichtung eine an sich bekannnte Einpunktkalibrierung durchgefüht. Da der intravasale Teil 11 der Sonde durch den Schutzschlauch 22 und den Reflektorkörper vor Kontamination geschützt ist, kann dies in unsteriler Umgebung erfolgen. Erst unmittelbar vor dem Einführen der Sonde wird die Schraubverbindung 12, 21 gelöst und der Schutzschlauch 22 und Reflektorkörper 25, 26 entfernt. Der intravasale Teil der Sonde 11 kann in einen bereits vorhandenen Katheter (z.B: zentralvenös) eingeführt und verschraubt werden. Durch den Anschluß 16 bleibt der Gefäßzugang erhalten.

## Patentansprüche

1. Kalibrierreflektorvorrichtung für eine faseroptische Sonde, aufweisend
- einen steifen Reflektorkörper (25, 26), welcher einen von einer Zugangsseite axial zugänglichen Sondenhohlraum zur Aufnahme eines Sondenendes (10) aufweist, sowie
- Anschlußmittel (12, 21) zum reversiblen Anschließen eines Anschlußstücks (13) an der Zugangsseite,
**dadurch gekennzeichnet, dass**
die Anschlußmittel (12, 21) in Schlußposition radial außerhalb eines Freiraums zur Sondendurchführung angeordnet sind, dessen Querschnittsausdehnung mindestens die Ausdehnung des Querschnitts des Sondenhohlraums besitzt.

2. Kalibrierreflektorvorrichtung gemäß Anspruch 1, wobei der Reflektorkörper eine steife, opake Einhüllung (26) und einen in die Einhüllung eingefügten Reflektoreinsatz (25), in welchem sich der Sondenhohlraum befindet, aufweist.

3. Kalibrierreflektorvorrichtung gemäß Anspruch 2, wobei die Einhüllung (26) zumindest innenseitig schwarz ist.

4. Kalibrierreflektorvorrichtung gemäß einem der Ansprüche 2-3, wobei der Reflektoreinsatz (25) eine transparente Kunststoffmatrix und einen Füllstoff aufweist.

5. Kalibrierreflektorvorrichtung gemäß Anspruch 4, wobei die Kunststoffmatrix Polyurethan und/oder Silikon aufweist.

6. Kalibrierreflektorvorrichtung gemäß einem der Ansprüche 4-5, wobei der Füllstoff Bariumsulfat aufweist.

7. Kalibrierreflektorvorrichtung gemäß einem der vorangehenden Ansprüche, wobei der Sondenhohlraum innenseitig eine strukturierte Oberfläche aufweist.

8. Kalibrierreflektorvorrichtung gemäß einem der vorangehenden Ansprüche, wobei die Anschlußmittel (12, 21) Verschraubungsmittel (12, 21) und/oder Bajonettanschlußmittel und/oder Einrastmittel und/oder Klemmittel aufweisen.

9. Kalibrierreflektorvorrichtung gemäß einem der vorangehenden Ansprüche, welche ein zwischen den Anschlußmitteln (12, 21) und dem Reflektorkörper angeordnetes Verlängerungsstück (22) aufweist.

10. Kalibrierreflektorvorrichtung gemäß Anspruch 9, wobei das Verlängerungsstück (22) zumindest abschnittweise biegsam ausgeführt ist.

11. Kalibrierreflektorvorrichtung gemäß einem der vorangehenden Ansprüche, wobei der Sondenhohlraum eine axiale Länge von mindestens dem dreißigfachen der Wurzel seiner Querschnittsfläche aufweist.

12. Kalibrierreflektorvorrichtung gemäß einem der Ansprüche 1-11, deren Sondenhohlraum zumindest einen Teil des biegsamen intravasalen Teils (11) einer faseroptischen Sonde aufnimmt, welche ein steifes Anschlußstück (13) aufweist, welches mittels der Anschlußmittel (12, 21) lösbar an die Kalibrierreflektorvorrichtung angeschlossen ist, wobei der intravasale Teil (11) der Sonde im Sondenhohlraum radiales Spiel hat.

13. Kalibrierreflektorvorrichtung gemäß Anspruch 12, wobei sich der intravasale Teil (11) der Sonde nur so weit in den Sondenhohlraum erstreckt, daß der Sondenhohlraum eine freie Länge von mindestens dem Zehnfachen der Wurzel seiner Querschnittsfläche aufweist.

14. Kalibrierreflektorvorrichtung gemäß Anspruch 13, wobei sich der intravasale Teil (11) der Sonde nur so weit in den Sondenhohlraum erstreckt, daß der Sondenhohlraum eine freie Länge von mindestens dem Zwanzigfachen der Wurzel seiner Querschnittsfläche aufweist.

15. Kalibrierreflektorvorrichtung gemäß einem der Ansprüche 12-14, wobei sich der intravasale Teil (11) der Sonde mindestens 20 mm und höchstens 60 mm in den Sondenhohlraum erstreckt.

16. Kalibrierreflektorvorrichtung gemäß einem der Ansprüche 12-15, wobei der intravasale Teil (11) der Sonde steril ist, und die Kalibrierreflektorvorrichtung abgedichtet ist, um die Sterilität des intravasalen Teils (11) der Sonde aufrechtzuerhalten.

17. Kalibrierreflektorvorrichtung gemäß einem der Ansprüche 12-16, wobei das Anschlußstück (13) der Sonde eine Spülöffnung aufweist.

18. Kalibrierreftektorvorrichtung gemäß einem der Ansprüche 12-17, welche eine hilfsmittelfrei öffenbare Blister-Umhüllung (1) aufweist.

19. Kalibrierreflektorvorrichtung gemäß Anspruch 18, wobei die Blisterumhüllung (1) eine Anreißstelle mit einer Aufreißhilfe (3) zum definierten Aufreißen der Blisterumhüllung (1) aufweist.

20. Kalibrierreflektorvorrichtung gemäß Anspruch 19, wobei die Sonde einen Steckverbinder (2) aufweist, welcher näher an der Anreißstelle plaziert ist, als der Sondenhohlraum.

21. Kalibrierreflektorvorrichtung gemäß Anspruch 20, wobei die Blisterumhüllung (1) eine Markierung aufweist, bis zu welcher die Blisterumhüllung (1) definiert aufreißbar ist dergestalt, daß der Steckverbinder (2) entnehmbar ist, zumindest der intravasale Teil (11) der Sonde jedoch in der Blisterumhüllung (1) verbleiben kann.

22. Verfahren zum sterilen Verpacken einer Kalibrierreflektorvorrichtung gemäß Anspruch 17, aufweisend folgende Schritte:
- Bereitstellen des steifen Reflektorkörpers,
- Einführen des freien Endes (10) des intravasalen Teils (11) der Sonde in den Sondenhohlraum,
- Herstellen einer lösbaren Verbindung des Anschlußstücks (13) mit dem Reflektorkörper,
- Plazieren der Kalibrierreflektorvorrichtung samt Sonde in eine Blisterumhüllung (1).
- Sterilisieren mit einem Sterilisationsgas,
- Verschließen der Blisterumhüllung (1).

## Claims

1. Calibration reflector device for a fiber-optic sensor, comprising
- a rigid reflector body (25, 26), which comprises a sensor cavity for accommodating a sensor end (10), which cavity is accessible from one access side, as well as
- connection means (12, 21) for reversibly connecting a connector piece (13) to the access side, **characterized in that**
the connection means (12, 21) are disposed radially outside a free space in the closed position, for passing the sensor through, the cross-sectional expanse of which space possesses at least the expanse of the cross-section of the sensor cavity.

2. Calibration reflector device according to claim 1, whereby the reflector body has a stiff, opaque sheathing (26) and a reflector insert (25) that is inserted into the sheathing, in which insert the sensor cavity is located.

3. Calibration reflector device according to claim 2, whereby the sheathing (26) is black at least on the inside.

4. Calibration reflector device according to one of claims 2-3, whereby the reflector insert (25) comprises a transparent plastic matrix and a filler.

5. Calibration reflector device according to claim 4, whereby the plastic matrix comprises polyurethane and/or silicone.

6. Calibration reflector device according to one of claims 4-5, whereby the filler comprises barium sulfate.

7. Calibration reflector device according to one of the preceding claims, whereby the sensor cavity has a textured surface on the inside.

8. Calibration reflector device according to one of the preceding claims, whereby the connection means (12, 21) comprise screw connection means (12, 21) and/or bayonet connection means and/or snap-in means and/or clamping means.

9. Calibration reflector device according to one of the preceding claims, which has an extension piece (22) disposed between the connection means (12, 21) and the reflector body.

10. Calibration reflector device according to claim 9, whereby the extension piece (22) is configured to be flexible at least in sections.

11. Calibration reflector device according to one of the preceding claims, whereby the sensor cavity has an axial length of at least thirty times the root of its cross-sectional area.

12. Calibration reflector device according to one of claims 1-11, wherein the sensor cavity accommodates at least a part of the flexible intravasal part of a fiber-optic sensor that has a rigid connector piece (13), that is connected releasably with the calibration reflector device by means of the connection means (12, 21), wherein the intravasal part (11) of the sensor has radial play in the sensor cavity.

13. Calibration reflector device according to claim 12, whereby the intravasal part (11) of the sensor extends into the sensor cavity only so far that the sensor cavity has a free length of at least ten times the root of its cross-sectional area.

14. Calibration reflector device according to claim 13, whereby the intravasal part (11) of the sensor extends into the sensor cavity only so far that the sensor cavity has a free length of at least twenty times the root of its cross-sectional area.

15. Calibration reflector device according to one of claims 12-14, whereby the intravasal part (11) of the sensor extends into the sensor cavity at least 20 mm and at most 60 mm.

16. Calibration reflector device according to one of claims 12-15, whereby the intravasal part (11) of the sensor is sterile, and the calibration reflector device is sealed, in order to maintain the sterility of the intravasal part (11) of the sensor.

17. Calibration reflector device according to one of claims 12-16, whereby the connector piece (13) of the sensor has a flushing opening.

18. Calibration reflector device according to one of claims 12-17, which has a blister pack (1) that can be opened without aids.

19. Calibration reflector device according to claim 18, whereby the blister pack (1) has a tear-open point having a tear-open aid (3) for tearing the blister pack (1) open in defined manner.

20. Calibration reflector device according to claim 19, whereby the sensor has a plug connector (2) that is placed closer to the tear-open point than the sensor cavity.

21. Calibration reflector device according to claim 20, whereby the blister pack (1) has a marking up to which the blister pack (1) can be torn open in defined manner, in such a manner that the plug connector (2) can be removed, but at least the intravasal part (11) of the sensor can remain in the blister pack (1).

22. Method for sterile packaging of a calibration reflector device according to claim 17, comprising the following steps:
- Making available the rigid reflector body that has a sensor cavity into which the intravasal part (11) of the sensor can be introduced with radial play,
- Inserting the free end (10) of the intravasal part (11) of the sensor into the sensor cavity,
- Producing a releasable connection of the connector piece (13) with the reflector body,
- Placing the calibration reflector device together with the sensor into a blister pack (1),
- Sterilizing with a sterilization gas,
- Closing the blister pack (1).

## Revendications

1. Dispositif réflecteur de calibration pour une sonde à fibres optiques, présentant :
- un corps de réflecteur rigide (25, 26), lequel présente une cavité de sonde accessible axialement par un côté d'entrée pour la réception d'une extrémité de sonde (10), ainsi que
- des moyens de raccord (12, 21) pour le raccord réversible d'une pièce de raccord (13) sur le côté d'entrée,
**caractérisé par le fait que** les moyens de raccord (12, 21) sont disposés en position de fermeture radialement à l'extérieur d'un espace libre pour le passage de sonde, dont la dimension en coupe transversale a au moins la dimension de la coupe transversale de la cavité de sonde.

2. Dispositif réflecteur de calibration selon la revendication 1, dans lequel le corps de réflecteur présente une enveloppe (26), opaque, rigide, et un insert de réflecteur (25) inséré dans l'enveloppe, insert de réflecteur dans lequel se trouve la cavité de sonde.

3. Dispositif réflecteur de calibration selon la revendication 2, dans lequel l'enveloppe (26) est noire au moins du côté intérieur.

4. Dispositif réflecteur de calibration selon l'une des revendications 2 et 3, dans lequel l'insert de réflecteur (25) présente une matrice en matière plastique transparente et une matière de charge.

5. Dispositif réflecteur de calibration selon la revendication 4, dans lequel la matrice de matière plastique présente du polyuréthane et/ou du silicone.

6. Dispositif réflecteur de calibration selon l'une des revendications 4 et 5, dans lequel la matière de charge présente du sulfate de baryum.

7. Dispositif réflecteur de calibration selon l'une des revendications précédentes, dans lequel la cavité de sonde présente du côté intérieur une surface structurée.

8. Dispositif réflecteur de calibration selon l'une des revendications précédentes, dans lequel les moyens de raccord (12, 21) présentent des moyens de vissage (12, 21) et/ou des moyens de fermeture à baïonnette et/ou des moyens d'encliquetage et/ou des moyens de collage.

9. Dispositif réflecteur de calibration selon l'une des revendications précédentes, lequel présente une pièce de rallonge (22) disposée entre les moyens de raccord (12, 21) et le corps de réflecteur.

10. Dispositif réflecteur de calibration selon la revendication 9, dans lequel la pièce de rallonge (22) est réalisée flexible au moins par segments.

11. Dispositif réflecteur de calibration selon l'une des revendications précédentes, dans lequel la cavité de sonde présente une longueur axiale d'au moins le trentuple de la base de sa surface transversale.

12. Dispositif réflecteur de calibration selon l'une des revendications 1 à 11, dont la cavité de sonde accueille au moins une partie de la partie intravasale flexible (11) d'une sonde à fibres optiques, laquelle présente une pièce de raccord rigide (13), laquelle est raccordée de façon détachable au dispositif réflecteur de calibration par les moyens de raccord (12, 21), la partie intravasale (11) de la sonde ayant un jeu radial dans la cavité de sonde.

13. Dispositif réflecteur de calibration selon la revendication 12, dans lequel la partie intravasale (11) de la sonde ne s'étend dans la cavité de sonde qu'aussi loin que la cavité de sonde présente une longueur libre d'au moins dix fois la base de sa surface transversale.

14. Dispositif réflecteur de calibration selon la revendication 13, dans lequel la partie intravasale (11) de la sonde ne s'étend dans la cavité de sonde qu'aussi loin que la cavité de sonde présente une longueur libre d'au moins vingt fois la base de sa surface transversale.

15. Dispositif réflecteur de calibration selon l'une des revendications 12 à 14, dans lequel la partie intravasale (11) de la sonde s'étend sur au moins 20 mm et au plus 60 mm dans la cavité de sonde.

16. Dispositif réflecteur de calibration selon l'une des revendications 12 à 15, dans lequel la partie intravasale (11) de la sonde est stérile, et le dispositif réflecteur de calibration est rendu étanche pour maintenir la stérilité de la partie intravasale (11) de la sonde.

17. Dispositif réflecteur de calibration selon l'une des revendications 12 à 16, dans lequel la pièce de raccord (13) de la sonde présente une ouverture de rinçage.

18. Dispositif réflecteur de calibration selon l'une des revendications 12 à 17, lequel présente une enveloppe - blister (1) apte à s'ouvrir sans moyen auxiliaire.

19. Dispositif réflecteur de calibration selon la revendications 18, dans lequel l'enveloppe blister (1) présente un emplacement de déchirure avec une aide de déchirure (3) pour la déchirure définie de l'enveloppe blister (1).

20. Dispositif réflecteur de calibration selon la revendication 19, dans lequel la sonde présente un connecteur à fiches (2), lequel est placé plus près de l'emplacement de déchirure que la cavité de sonde.

21. Dispositif réflecteur de calibration selon la revendication 20, dans lequel l'enveloppe de blister (1) présente un marquage jusqu'auquel l'enveloppe blister (1) est déchirable de façon définie de telle manière que le connecteur à fiches (2) peut être retiré, mais qu'au moins la partie intravasale (11) de la sonde peut rester dans l'enveloppe blister (1).

22. Procédé pour réaliser un emballage stérile d'un dispositif réflecteur de calibration tel que défini à la revendication 19, présentant les étapes suivantes :
- préparation du corps de réflecteur rigide ;
- introduction de l'extrémité libre (10) de la partie intravasale (11) de la sonde dans la cavité de sonde ;
- fabrication d'une liaison détachable de la pièce de raccord (13) avec le corps de réflecteur ;
- mise en place du dispositif réflecteur de calibration avec la sonde dans une enveloppe blister (1) ;
- stérilisation par un gaz stérilisant ;
- fermeture de l'enveloppe blister (1).
